# EUROPEAN PATENT APPLICATION

(11) **EP 4 567 831 A1**
(43) Date of publication of application: **11.06.2025**
(21) Application number: 23215357.7
(22) Date of filing: 08.12.2023
(51) Int. Cl.: G16H 40/63, G16H 40/67, G16H 50/50

(54) **SYSTEMS AND METHODS FOR CUSTOMISABLE MEDICAL TRAINING**

(71) Applicant: Simlogic AB, 41390 Göteborg (SE)
(72) Inventor: OLSEN, Ole Johan, 41390 Göteborg (SE); LIM, Han Lea, 41390 Göteborg (SE)
(74) Representative: Barker Brettell Sweden AB

(57) **Abstract**

A customisable medical training system includes a processor and a memory, with the memory containing instructions that cause the processor to generate or modify a virtual environment based on a customisation input. The virtual environment comprises a 3D virtual scene displaying a virtual patient, a state of the virtual patient, and a set of one or more available virtual medical resources displayable in the 3D virtual scene. The system communicates with a VR display system to render the generated or modified virtual environment to a training recipient via the VR display system. The customisation input is a manual input received to customise the state of the virtual patient and/or the set of available virtual medical resources.

## Description

### Field of Invention

The technology relates to the field of medical training, specifically focusing on the use of virtual reality (VR) systems to create immersive and customizable virtual training environments for healthcare professionals.

### Background

It is well known that providing training to medical professionals (doctors, surgeons, anaesthetists and other clinicians, as well as nurses, technicians and support staff) is very important for a variety of reasons. Healthcare professionals have ethical and legal obligations to their patients, and patients who place their trust in these professionals ought to feel confident that these obligations will be met. Many medical procedures involve clinicians facing several critical (sometimes life-or-death) decisions that require sound clinical judgement. It is essential for patient safety and wellbeing that the clinicians have developed this judgement to the extent necessary to make such decisions under pressure and to diagnose and treat patients accurately. Failure to adequately train medical professionals can lead to serious harm and adverse consequences.

Moreover, medicine is a highly complex and rapidly evolving field in which the latest treatments, technologies and research are continuously advancing. Even a surgeon who has been trained very thoroughly and extensively will continue to require ongoing training and education to stay up to date with these advancements, both for the good of their patients and for their own professional growth and development. This particularly so in the case of specialist fields of medicine, which may require unique skills and experience.

Many procedures are performed by healthcare professionals working in a team, and often an interdisciplinary team, rather than as individuals. This brings its own unique set of challenges and opportunities for growth and development even highly skilled professionals cannot hope to deliver the highest possible standard of care unless they can communicate effectively and collaborate with one another. Present technology for training of healthcare professionals is undesirably limited in its scope, functionality, availability, affordability, realism, immersiveness and power, particularly in relation to developing communication, collaboration and teamwork skills.

In the current state of the art, it is typical to train healthcare professionals by sending them to a specialised facility known as a medical or surgical "simulation centre" (also sometimes referred to as a "skills lab" or "training centre"), in which hands-on physical apparatus and/or models are provided for clinicians to replicate and practice various medical procedures (including e.g., laparoscopic, endoscopic, or open surgical procedures). These simulation centres can provide a range of resources to facilitate training, including (among other resources):
i) mock operating rooms or surgical suites designed to resemble actual clinical settings, complete with surgical tables, lighting, anaesthesia equipment, and the like;
ii) a range of medical/surgical devices, tools, products and equipment (which may either be genuine articles suitable for actual use in surgery, or may be modified "mock"/"practice" articles that have been adapted for training purposes e.g., by removing sharp blades, staples, electrosurgical functionality, and so forth); and
iii) simulated "patients", in the form of high-fidelity lifelike manikins or anatomical models that replicate human body structures, to allow trainees to practice surgical procedures and techniques.

As popular as these simulation centres are, they are not without their drawbacks. The requisite surgical equipment can be highly expensive to obtain (even for "mock" instruments and apparatus used solely for training purposes), and ongoing costs can be high in the case of training that involves the use of expendable or disposable physical resources (such as surgical staples or other fastening/sealing members, surgical suture cartridges, irrigation fluid, battery packs, and so forth). As far as patient simulators are concerned, even the simplest and most affordable offerings in the market are currently very expensive due to the inherent difficulty of using electromechanical means to mimic the look and feel of a human being. In addition, the manikins usually require moulage (replica blood, fluids, and so forth) in order for their simulated injuries to appear realistic. Accordingly, these manikins typically must also be processed (e.g., cleaned) in between every successive use, which adds to the expense, complexity, and duration of these training sessions. It would be preferable to provide training means in which simulated injuries can be created and repeated more easily.

Another problem is that these simulation centres require all persons undertaking the training session to be assembled in the same physical location simultaneously. Due to constraints imposed by geography, professionals' schedules and other commitments, transport availability/unavailability (e.g., rail strikes), this diminishes the total number of suitable opportunities on which training can be arranged. Moreover, since only one team of trainees can use the same room and equipment at any one given time, a shortage of supply is created, driving up the expense of training.

The training offered frequently takes the form of a specific invented surgical scenario revolving around a particular set of desired learning outcomes or objectives for clinical staff. For example, one scenario may involve examining, assessing and managing a penetrating abdominal injury (stab injury) according to Advanced Trauma Life Support (ATLS)/Definitive Surgical Trauma Care (DSTC) guidelines whilst considering bleeding and vascular access. Another may involve the diagnosis and handling of pneumothorax in a patient with partial airway obstructions. There may be a list of available scenarios/courses that have each been uniquely designed by the training provider. The hospital or other medical facility arranging the external training of its staff at an off-site simulation centre has to make a selection from among the available courses/scenarios/environments based on which most closely aligns with whatever specific intended training goal that they have in mind. International certification courses such as ATLS, ACLS (Advanced Cardiovascular Life Support), Basic Life Support (BLS) and the like are courses involving hands-on simulations conducted at designated simulation centres. If the simulation centre doesn't offer a specific scenario, or it only offers a version of the scenario which is suboptimal in some way, then the only options are either to select the "next-best" offering, to look for an alternative external provider of training, or to spend time and money developing their own training facilities and/or conducting their own custom simulations. The hospital or other medical facility rarely has any ability to control the content or course of any one training scenario of an external provider, and have to rely on the simulation centre providing an acceptably varied and complete range of scenarios.

The present inventors have identified that there is a need to improve upon current systems and methods for medical training, particularly for the training of teams. However, due to how ubiquitous and well-established current training practices are, new developments in the field of training for teams of medical professionals are likely to be rejected if they are not to some extent familiar, recognisable or backwards-compatible - that is, it is an aim of the present invention to build upon and improve the current paradigm by which teams of medical staff train, rather than to overhaul and replace it completely.

Very recently, a limited number of commercial entities have begun to develop software for training medical professionals using virtual reality (VR) systems. VR technology provides a computer-generated three-dimensional virtual environment, simulating the effect of being physically present within a virtual scene. Typically this is achieved by using a head-mounted display (HMD) comprising displays for each eye of a user to provide a 3D visual experience and sensors that track head movements, thus allowing users to look around and move around (e.g., walk around) in the simulated virtual environment. Input devices such as handheld controllers can allow the user to interact with the virtual environment.

Though a welcome development, these VR offerings still fall short of ideal, and in many cases still suffer from a lot of the same drawbacks associated with traditional clinical training resources and methodologies. Accordingly, it would be advantageous to provide VR-based medical training systems and methods that improve upon the present state of the art.

### Summary

According to a first aspect of the disclosure, a customisable medical training system is provided, comprising a processor and a memory. The memory contains instructions which, when executed by the processor, cause the processor to generate or modify a virtual environment based on a customisation input. This virtual environment includes a 3D virtual scene with a virtual patient, a state of the virtual patient, and a set of one or more available virtual medical resources displayable in the 3D virtual scene. The system communicates with a VR display system to render the generated or modified virtual environment to a training recipient via the VR display system. The customisation input is a manual input received to customise the state of the virtual patient and/or the set of available virtual medical resources. This aspect allows for tailored medical training scenarios based on the specific needs of the training recipient.

Optionally in some examples, generating or modifying the virtual environment involves modifying an existing virtual environment rendered to the training recipient. This allows for real-time adjustments to the training scenario as needed.

Optionally in some examples, the VR display system is a first VR display system, and the existing virtual environment is a virtual environment rendered to a supervising user via a second VR display system in communication with the processor. This enables the supervising user to monitor and adjust the training scenario as needed.

Optionally in some examples, generating or modifying the virtual environment involves generating the virtual environment as a new virtual environment. This allows for the creation of entirely new training scenarios based on the customisation input.

Optionally in some examples, the customisation input is provided via an interaction with a GUI, such as an interaction made outside of the virtual environment, an interaction with an application running on a computing device, an interaction with a web form, or a selection from a menu. This provides an easy and intuitive way for the supervising user to customise the virtual environment.

Optionally in some examples, the customisation input is provided via an interaction of the supervising user with at least a component of the VR display system. This allows for seamless integration of the customisation process within the virtual environment.

Optionally in some examples, rendering the virtual environment to the training recipient involves setting the external appearance of the virtual patient based on the state of the virtual patient. This provides a realistic and immersive training experience for the training recipient.

Optionally in some examples, rendering the virtual environment to the training recipient involves setting the behaviour of the virtual patient based on the state of the virtual patient. This allows for a more dynamic and engaging training experience.

Optionally in some examples, the set behaviour is a behaviour of the virtual patient in response to a given interaction of the training recipient with the virtual patient. This provides a realistic and responsive training environment.

Optionally in some examples, rendering the virtual environment to the training recipient involves setting the behaviour of a virtual medical resource in the set of available virtual medical resources based on the state of the virtual patient. This allows for a more accurate and realistic simulation of medical resource usage in the training scenario.

Optionally in some examples, the set behaviour is a behaviour of the virtual medical resource in response to a given interaction of the training recipient with the virtual patient. This provides a more interactive and engaging training experience.

Optionally in some examples, the given interaction involves the use of a virtual medical resource in the set of available virtual medical resources within the virtual environment by the training recipient upon the virtual patient. This allows for hands-on training experience with various medical resources.

Optionally in some examples, the virtual environment further includes a set of one or more available digital audio assets configured to be played in the virtual environment to the training recipient. This enhances the realism and immersion of the training experience.

Optionally in some examples, rendering the virtual environment to the training recipient involves causing or enabling the training recipient to interact with the virtual environment to cause one of the digital audio assets to be played in the virtual environment to the training recipient. This provides an interactive and engaging audio experience for the training recipient.

Optionally in some examples, rendering the virtual environment to the training recipient involves causing the display of one or more virtual medical resources of the set of available virtual medical resources in the 3D virtual scene. This provides a wide range of resources for diverse training scenarios.

Optionally in some examples, rendering the virtual environment to the training recipient involves enabling the training recipient to interact with the virtual environment to cause the display of one or more virtual medical resources of the set of available virtual medical resources in the 3D virtual scene. This allows for a more interactive and engaging training experience.

Optionally in some examples, the interaction by the training recipient with the virtual environment is by means of the VR display system. This provides a hands-on training experience with the virtual medical resources.

Optionally in some examples, the displayed one or more virtual medical resources include a virtual medical item, a virtual medical tool, a virtual display, a virtual consumable medical product, a virtual pharmaceutical product, a 2D media asset, or a text asset. This provides a wide range of resources for diverse training scenarios.

Optionally in some examples, rendering the virtual environment to the training recipient involves displaying or enabling the training recipient to cause the display of a 2D media asset via a virtual display in the 3D virtual scene. This allows for the integration of additional educational materials within the virtual environment.

Optionally in some examples, rendering the virtual environment to the training recipient involves displaying or enabling the training recipient to cause the display of a text asset via a virtual display in the 3D virtual scene. This allows for the integration of customised text within the virtual environment to simulate e.g., results of laboratory tests.

Optionally in some examples, the training recipient is a first training recipient, the VR display system is a first VR display system, and the instructions are further configured to cause the processor to communicate with a third VR display system to render the generated or modified virtual environment to a second training recipient via the third VR display system. This allows for simultaneous training of multiple recipients in the same virtual environment.

Optionally in some examples, the customisation input is a first customisation input received from a first supervising user, and the instructions are further configured to cause the processor to receive a second customisation input from a second supervising user, modify the virtual environment based on the second customisation input, and communicate with the VR display system to render the generated or modified virtual environment to the training recipient via the VR display system. This allows for collaborative customisation and supervision of the training scenario by multiple supervising users.

Optionally in some examples, the state of the virtual patient includes one or more of body temperature, heart rate, respiration rate, blood pressure, oxygen saturation, blood glucose level, blood CO₂ level, end-tidal CO₂ level, age, gender, ethnicity, position, clothing state, responsivity, pupil size, pupil reactivity, skeletal pathology, respiratory pathology, or circulatory pathology. This allows for a highly customisable and realistic virtual patient for tailored training scenarios.

According to a second aspect of the disclosure, a VR display system is provided, comprising a processor and a memory. The memory contains instructions which, when executed by the processor, cause the processor to communicate with a customisable medical training system and render a virtual environment generated or modified by the customisable medical training system based on a customisation input to a training recipient. The virtual environment includes a 3D virtual scene with a virtual patient, a state of the virtual patient, and a set of one or more available virtual medical resources displayable in the 3D virtual scene. This aspect allows for an immersive and interactive training experience for the training recipient.

According to a third aspect of the disclosure, a computer-implemented method of providing customised medical training is provided. The method includes generating or modifying a virtual environment by a processor based on a customisation input, communicating with a VR display system, and rendering the generated or modified virtual environment to a training recipient by the VR display system. The customisation input is a manual input received to customise the state of the virtual patient and/or the set of available virtual medical resources. This method allows for tailored medical training scenarios based on the specific needs of the training recipient.

According to a fourth aspect of the disclosure, a non-transitory computer-readable medium is provided, comprising instructions which, when read by a computer, cause the computer to generate or modify a virtual environment based on a customisation input and communicate with a VR display system to render the generated or modified virtual environment to a training recipient via the VR display system. The customisation input is a manual input received to customise the state of the virtual patient and/or the set of available virtual medical resources. This aspect allows for the implementation of the customisable medical training system on various computing devices.

The present invention allows a supervising user (e.g., a trainer) to customise aspects of a scenario (such as the patient's state), this customisation being reproduced in VR to at least one user and optionally a plurality of users. Each of the users may be simultaneously performing their own surgical tasks in the virtual environment using the virtual medical resources (e.g., tools and equipment). With each task performed, the virtual patient, medical resource, scene, or environment itself may generate a corresponding response, e.g., by displaying a certain behaviour. The patient's state can be modified by a supervising user either from within the virtual environment (using a VR display device) or by using non-VR means, such as an additional computing device acting as a "trainer station", and in either case such modifications can be reproduced instantaneously in VR to all users. For example, on one hand, if a training recipient administers a virtual drug to the virtual patient then there may be a corresponding increase in chest movement visible to all of the training recipients present in the virtual environment, and/or an alert sound may be played to indicate a deterioration in the virtual patient's vital signs. Equally, however, one or both of these effects may result as a direct result of the supervising user's interaction to modify the patient state. As described herein, the present invention enables the customisation of a plurality of different aspects of patient state; such a comprehensive degree of customisability is not known from any simulation hardware or software in the art at present.

### Brief Description of the Drawings

Examples are described in more detail below with reference to the appended drawings.
Figure 1 is a block diagram illustrating components of or relating to a customisable medical training system.
Figure 2 is a block diagram illustrating a VR display system and components thereof.
Figure 3 is a block diagram illustrating elements and properties of a virtual environment for use in medical training.
Figures 4a, 4b and 4c show an exemplary graphical user interface for use in generating a customised virtual environment based on a customisation input.
Figure 5 shows an exemplary graphical user interface for use in modifying a virtual environment based on a customisation input.
Figure 6 is an exemplary view seen by a training recipient.
Figure 7 is an exemplary view seen by a supervising user.
Figure 8 is a flowchart illustrating a method of providing customised medical training.

### Detailed Description

The detailed description set forth below provides information and examples of the disclosed technology with sufficient detail to enable those skilled in the art to practice the disclosure.

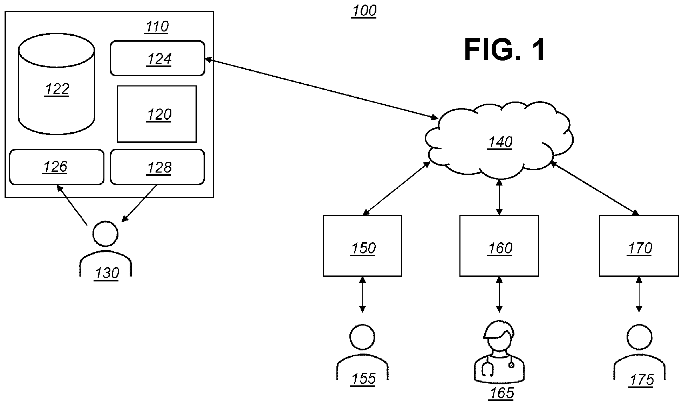

Figure 1 shows a block diagram 100 illustrating components of or relating to an exemplary customisable medical training system 110. The system 110 includes a processor 120 and a memory 122 containing instructions that, when executed by the processor, cause the processor to generate or modify a virtual environment based on a customisation input. The system further includes a communication interface 124 configured to communicate over a network 140 (e.g., a LAN, or a wide-area network such as the internet), a user input device 126, and a display 128 configured to present a graphical user interface to a user 130. Also shown in Figure 1 are VR display systems 150, 160 and 170 for rendering the generated or modified virtual environment to training recipients 155, 175 and supervising user 165, each VR display system configured to communicate over network 140.

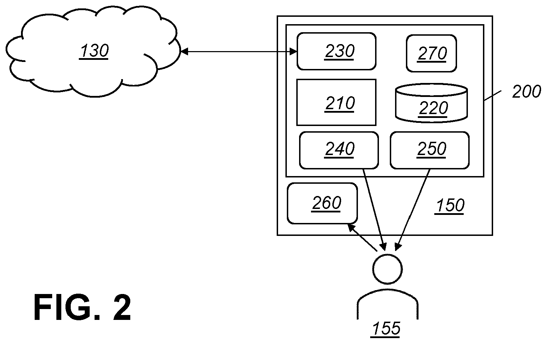

Figure 2 is a block diagram illustrating an exemplary VR display system 150 and exemplary components thereof. The VR display system 150 includes a head-mounted-device (HMD) or headset 200 comprising a processor 210, a memory 220, a communication interface 230 configured to communicate over network 140, a display 240, an audio device 250 (e.g., a speaker), and a motion sensor 270. VR display system 150 further includes one or more input devices 260, such as a handheld controller or a pair of handheld controllers allowing user 155 to interact with the virtual environment.

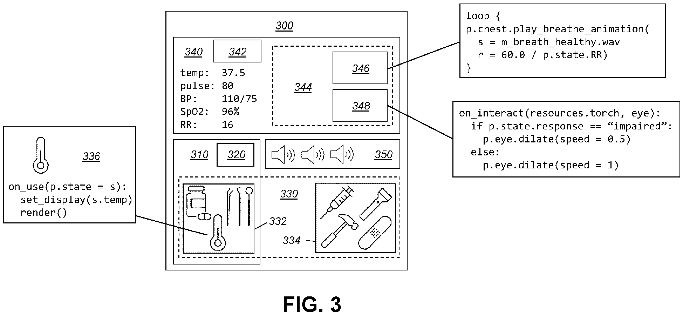

Figure 3 is a block diagram illustrating elements and properties of an exemplary virtual environment 300 for use in medical training. The virtual environment 300 includes a 3D virtual scene 310 with a virtual patient 320, a state 340 of the virtual patient, and a set of available virtual medical resources 330 displayable in the 3D virtual scene. The illustrated set of available virtual medical resources 330 comprises a set of displayed virtual medical resources 332 that are present in 3D virtual scene 310, and a set of non-displayed virtual medical resources 334 which, though potentially displayable, are not currently present in 3D virtual scene 310. Each virtual medical resource may optionally be associated with one or more virtual resource behaviours 336. Patient state 340 further comprises an external appearance 342 of the virtual patient (which may be based on e.g., a gender, an age, and/or one or more pathologies/injuries), and a set of one or more behaviours 344 of the virtual patient, including one or more passive behaviours 346 and/or one or more responsive behaviours 348. The illustrated exemplary virtual environment 300 further comprises a set of digital audio assets 350.

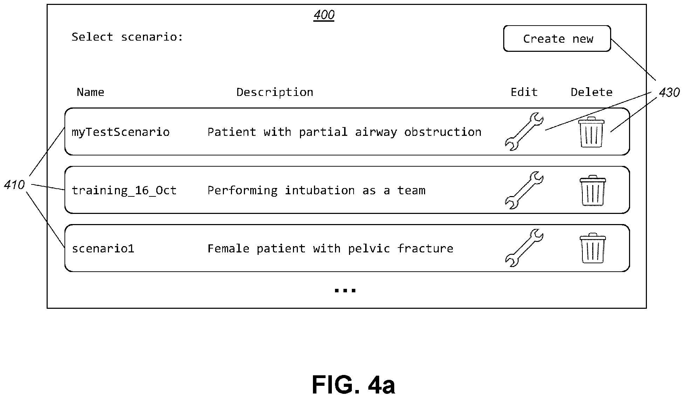

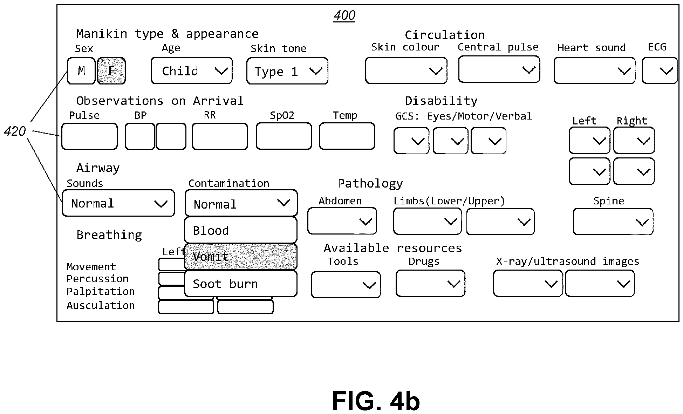

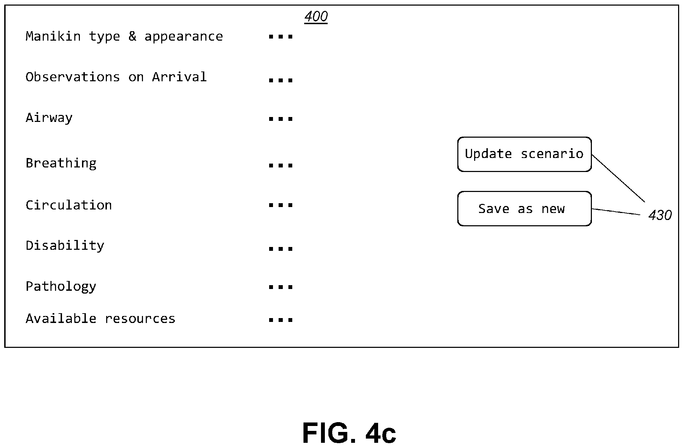

Figures 4a, 4b, and 4c show an exemplary graphical user interface 400 for use in generating or modifying a customised virtual environment 410 based on a customisation input. The interface includes various input fields 420 and options for customising the state of the virtual patient and the set of available virtual medical resources. The interface further comprises control means 430 for editing, saving, copying and deleting virtual environments.

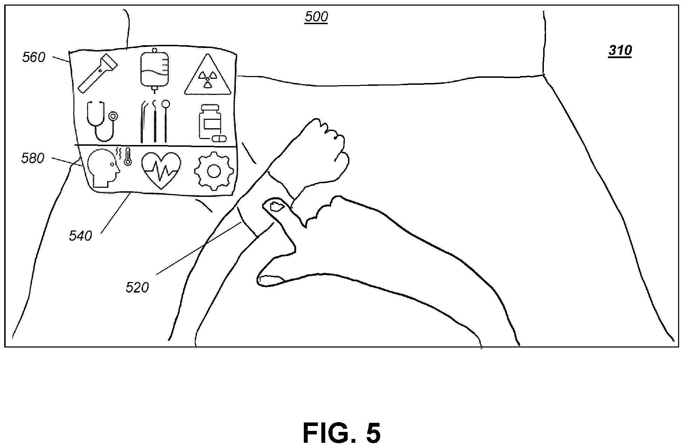

Figure 5 shows an exemplary graphical user interface 500 for use in modifying a virtual environment based on a customisation input. The interface is a modified version of the interface which would be seen by a standard training recipient 155, 175 using a VR display system 150, 170 with medical training system 110. In contrast with this standard interface, interface 500 is configured for use by a supervising user 165 and provides additional functionality via which a customisation input can be provided.

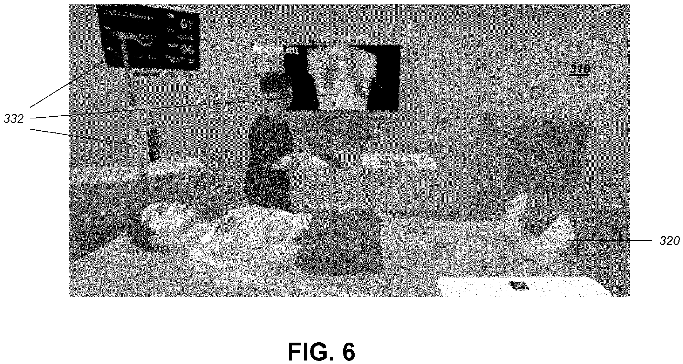

Figure 6 is an exemplary view seen by a training recipient. The view is a view of a 3D virtual scene 310 with a virtual patient 320 and various displayed virtual medical resources 332 available for interaction.

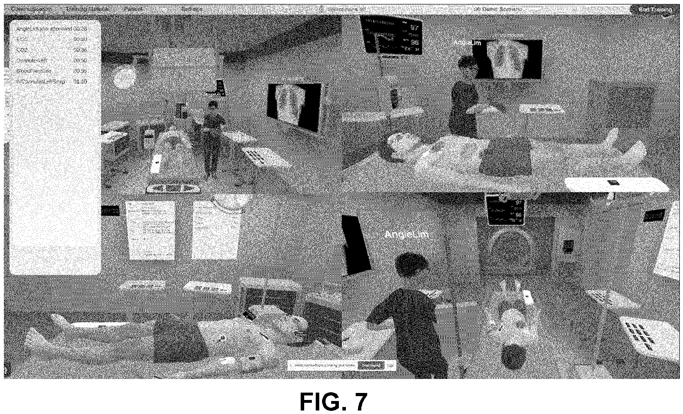

Figure 7 is an exemplary view seen by a supervising user. The view comprises a plurality of views of 3D virtual scene 310 with a virtual patient 320 and various virtual medical resources 332 available for interaction, as well as a supervisory interface 700 for managing, monitoring, modifying and observing the virtual environment.

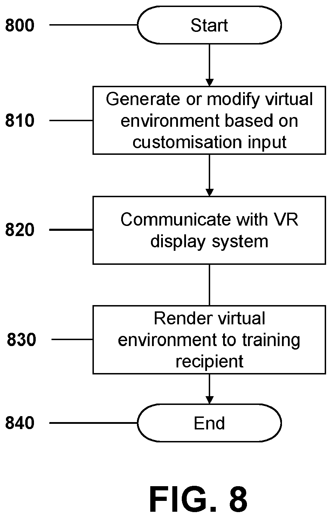

Figure 8 is a flowchart illustrating a method of providing customised medical training. The method starts at step 800 and includes generating or modifying 810 a virtual environment based on a customisation input, communicating 820 with a VR display system, and rendering 830 the generated or modified virtual environment to a training recipient. The method ends at step 840.

### 2. System and Component Details

Details of the customisable medical training system, the VR display system, and the networking and other components that make up the overall system will now be discussed with reference to Figs. 1 and 2.

### 2.1. Customisable Medical Training System

Referring first to Fig. 1, customisable medical training system 110 comprises a processor 120 and a memory 122, which contains instructions that, when executed by the processor, cause the processor to generate or modify a virtual environment based on a customisation input. Processor 120 may comprise a central processing unit (CPU), as manufactured by Intel, AMD, ARM and the like and as are well known to those of ordinary skill in the art, and may use any suitable CPU architecture (e.g., x86, MIPS, and so forth). Processor 120 may comprise a microcontroller, a field-programmable gate array (FPGA), a control circuit (such as an application-specific integrated circuit (ASIC)), or any other suitable electronic processing means. Though depicted as a single element in Fig. 1, processor 120 may comprise a plurality of processors, for example as a CPU with multiple cores or as a distributed processing architecture. Memory 122 may comprise any volatile or non-volatile memory suitable for providing temporary or long-term storage for data used by processor 120, such as a random access memory (RAM), flash memory (e.g., one or more USB drives, solid state drives or memory cards), read-only memory, hard disk drive, floppy disk, cache, or virtual memory. Though depicted in Fig. 1 as a single element, memory 122 may in practice comprise a plurality of memory devices e.g., a plurality of RAM devices, a RAID, or a distributed memory architecture. System 110 may operate substantially as a general purpose computer running an operating system and fetching/caching/executing computer-readable instructions as well known in the art.

The customisation input may be a manual input received to customise the state of the virtual patient and/or the set of available virtual medical resources. In some examples, the manual input may be provided by user input device 126. User input device 126 may comprise any suitable input means for the medical training system 110 including but not limited to one or more keyboards, mice, touchscreens, trackpads, trackballs, haptic feedback devices, cameras and/or microphones. Medical training system 110 may be configured to process and/or convert various inputs using processor 120, for example by processing hand-drawn or stylus-drawn inputs to convert them into textual input, by processing audio data captured by a microphone (e.g., speech) to convert it into textual input, by applying image processing to perform gesture/object recognition, and so forth. User input device 126 advantageously enables user 130 to provide a customisation input for generating or modifying a virtual environment. However, user input device 126 is merely an optional feature of the invention rather than an essential one, because the customisation input may instead be provided by another user (such as supervising user 165) via separate means.

Medical training system 110 may optionally further comprise a communication interface 124 configured to communicate over a network 140. Communication interface 124 may comprise any one or more hardware or software components facilitating the exchange of data and information and acting as an intermediary to transmit, receive, and interpret said data in a consistent and standardized manner, such as an ethernet, Wi-Fi, serial communication, cellular communication, TCP/IP, and so forth. Advantageously, communication interface 124 can enable the delivery of information relating to the generated or customised virtual environment in real time, and can enable the rendering of the generated or customised virtual environment to users 155, 165, 175 in real time, providing immersive interaction between each user and virtual environment.

Medical training system 110 may optionally further comprise a display 128 configured to present a graphical user interface to a user. Display 128 may comprise any suitable display device, screen or monitor (such as any known LCD, OLED, CRT and/or touchscreen device), and may be configured to present a GUI (such as that disclosed in relation to Figs. 4a-4c) to user 130. Display 128 may be configured to present one or more menus, applications, forms, web browsers and the like to user 130. Display 128 advantageously improves the ease with which user 130 can provide a customisation input for generating or modifying a virtual environment via user input device 126, where such a user input device is present. Additionally or alternatively, irrespective of whether user input device 126 is present, display 128 can advantageously be configured to present oversight/monitoring information to user 130 about the virtual environment and its state, and/or about users 155, 165, 175 and their behaviour in the 3D virtual scene. However, display 128 is merely an optional feature of the invention rather than an essential one, because the customisation input may instead be provided by another user (such as supervising user 165) via separate means, and supervising user 165 may be responsible for monitoring/oversight of the virtual environment as training progresses.

During active interaction of one or more users 155, 165, 175 with the virtual environment via their respective device (150, 160, 170) - in other words, whilst the VR simulation is ongoing - the virtual environment (and possibly, though not necessarily, some or all of the digital assets therein) may optionally be hosted by an external server (not shown) connected to network 140. The host server can be configured to store information about the virtual environment and perform operations to cause the virtual environment to be presented to one or more of the users 155, 165, 175 via their respective VR display device(s). The host server may further be configured to receive input from the behaviour of the users whilst the simulation is ongoing (e.g., details of the users' movements and actions as they explore the 3D virtual scene and interact with virtual medical resources displayed in the virtual environment) and, in response to these inputs, update the state of the simulation and transmit updated information to the users in response accordingly. For example, if a first user 155 interacts with VR display device 150 in a way indicative of moving from one position in the 3D virtual scene to another, information representing this movement can be transmitted to the hosting server, which then updates that user's position in the memory of the host server and transmits information representing the new position to VR display devices 160, 170. As a consequence, users 165 and 175 viewing the scene via VR display devices 160 and 170 perceive user 155 as having moved to the new position.

The data and information necessary to simulate the virtual environment may be distributed between the host server and respective VR devices 150, 160, 170. As will be appreciated by those of ordinary skill in the art, latency and responsiveness as perceived by a user can be improved by increasing the amount of data that is stored locally (e.g., cached) on their VR display device, with the trade-off of such caching including (though not necessarily limited to an increase in memory required).

In one exemplary configuration, for instance, VR display device 150, 160, 170 may locally store/cache graphics and virtual environment data (3D models, textures, assets), user position and orientation data, sound and audio files/effects, and local game state. This can ensure smooth and responsive rendering without the need for constant streaming from the host server, minimise head tracking latency, reduce audio lag and ensure the user's actions (e.g., picking up a virtual medical resource) are reflected immediately to them. In optional (though non-essential) implementations, the VR display device may also perform some physics and collision detection calculations locally. The VR display device may enforce at least some of the logic and rules of the simulation (e.g., virtual patient and virtual medical resource behaviours as dictated by the virtual patient's internal state and pathologies) locally. Conversely, the host server may maintain the canonical global state of the virtual environment (including positions of all users and virtual medical resources), enforce the logic and rules of the simulation, manage network latency and synchronisation, and so forth. Of course, other configurations based on a variety of different computational hardware, software and/or network constraints will be readily apparent to those skilled in the art, and arrangements for communication and storage of resources between the host server and VR display devices can be modified according to the parameters of any specific use case as appropriate. In a preferred embodiment, the digital assets for the set of displayable virtual medical resources are downloaded to each VR display device in advance of the virtual environment being rendered/in advance of the simulation commencing - for example, digital assets or files may be downloaded as part of downloading a VR application ("app") to the VR display device, with the hosting server merely sending information concerning how to use, render, or play the downloaded assets during the simulation (i.e., in the virtual medical procedure). This can lead to reduced latency, reduced congestion and reduced bandwidth consumption for the network when the environment is being rendered.

### 2.2. VR Display System

As depicted in Fig. 2, VR display system 150 comprises a head-mounted device (HMD) 200 and an optional input device 260. The HMD comprises a CPU or processor 210, a memory 220, a motion sensor 270, a display 240, an audio output system in the form of a speaker 250, and a communication interface 230 configured to establish connections and communicate over network 130.

Processor 210 may be configured to render 3D virtual scenes and environments, manage real-time user interactions, facilitate data processing and ensure a seamless and responsive VR experience. Processor 210 may comprise a central processing unit (CPU), as manufactured by Intel, AMD, ARM and the like and as are well known to those of ordinary skill in the art, and may use any suitable CPU architecture (e.g., x86, MIPS, and so forth). Processor 120 may comprise a microcontroller, a field-programmable gate array (FPGA), a control circuit (such as an application-specific integrated circuit (ASIC)), or any other suitable electronic processing means. Though depicted as a single element in Fig. 2, processor 120 may comprise a plurality of processors, for example as a CPU with multiple cores or as a distributed processing architecture.

Memory 220 may be configured for storing instructions executable by processor 210 to cause processor 210 to perform any one or more of the above processing operations. Memory 220 may further optionally be configured for storing VR software, textures, 3D models, and associated data, allowing rapid data access, thereby minimizing latency and supporting uninterrupted interaction with the virtual environment as described hereinabove. Memory 220 may comprise any volatile or non-volatile memory suitable for providing temporary or long-term storage for data used by processor 210, such as a random access memory (RAM), flash memory (e.g., one or more USB drives, solid state drives or memory cards), read-only memory, hard disk drive, floppy disk, cache, or virtual memory. Though depicted in Fig. 2 as a single element, memory 220 may in practice comprise a plurality of memory devices.

The optional motion sensor 270 may be provided and configured to monitor the head movements of user 155. The sensor translates physical head movements into simulated motion of the user's viewpoint within the 3D virtual scene, allowing the user to freely explore and interact with the virtual environment. In one example, motion sensor 270 may comprise an Inertial Measurement Unit (IMU) incorporating one or more accelerometers and/or gyroscopes. Motion sensor 270 advantageously provides greater immersion in the virtual scene and environment, although is not an essential feature since control of the viewpoint can be provided in the alternative by other means such as input device(s) 260.

Display 240 provides a visual interface with the virtual environment, and can comprise at least a first screen arranged in HMD 200 so as to be positioned in front of the user's left eye during use of system 150 and at least a second screen arranged in HMD 200 so as to be positioned in front of the user's right eye during use of system 150, the first and second screen thereby rendering stereoscopic 3D imagery and provide a convincing an immersive virtual environment. Preferably the screen or screens are high-resolution, in order to reduce motion sickness experienced by the user.

An optional audio device 250 (e.g., comprising a speaker) may be provided as an element of system 150 or HMD 200, advantageously complementing the visual element of the virtual environment and providing enhanced realism. Audio device 250 may be suitable and/or configured to output sound based on digital audio assets relating to the 3D virtual scene itself (e.g., spatial sound, immersive audio, soundscapes, environmental sound, and so forth); to the virtual patient (e.g., heartbeat sounds, dialogue lines, pain indications, and so forth); to the virtual medical resources (e.g., beeping produced by cardiac monitoring apparatus, individual sounds associated with use of virtual medical equipment, sound present in 2D media (e.g., video) assets played on a virtual display, and so forth); and/or to the user's movements and interactions within the virtual environment (footsteps, voice communication with other users/supervising users, and so forth).

Communication interface 230 can advantageously facilitate connectivity to the network 130, supporting real-time interaction with the virtual environment (and optionally the other users), downloading of assets and content, and updating of software. Communication interface 230 may comprise any one or more hardware or software components facilitating the exchange of data and information and acting as an intermediary to transmit, receive, and interpret said data in a consistent and standardized manner, such as an ethernet, Wi-Fi, serial communication, cellular communication, TCP/IP, and so forth, contingent on the HMD's design and technological specifications.

Input device 260 serves as a means by which user 155 can interact with the virtual environment. Exemplary input devices may include, but are not limited to, handheld controllers, gloves or haptic feedback devices, gesture recognition technology, and voice control mechanisms. These elements enable users to manipulate virtual objects and engage with the virtual world using a variety of input methods, including physical gestures, tactile feedback, and voice commands. Input device 260 may be a single handheld controller configured to be held in one hand, or a single handheld controller configured to be held with both hands together, or a pair of handheld controllers each of which is configured to be held in one hand (left and right respectively). Input device 260 may comprise a wand or rod controller. Input device 260 may comprise VR gloves or other tracking means, such as one or more cameras or other sensors configured to track motions and gestures made with the hands and/or body (e.g., reaching, grabbing, waving, pointing, squeezing, and the like). Such cameras/sensors may be components built into HMD 200 itself. Input device 260 may be configured to provide haptic feedback. In addition to user input being provided via VR-specific means such as VR controllers or hand tracking hardware/software, user input can be provided via non-VR-specific means such as traditional mouse, keyboard or trackpad controls, or via touch or motion-based controls input using an application running on a user's mobile device. In a preferred implementation of the present invention, user input device 260 is a hand tracking means configured to detect movements and gestures made by user 155 using their hands.

In operation, user 155 dons HMD 200, immersing them within the virtual environment. As the user moves their head, motion sensor 270 accurately tracks these movements. Display 240 (and optionally audio device 250) deliver visual (and auditory) feedback, producing an effect on user 155 at the sensory level (and not merely a cognitive level), in the form of a sensation of their real physical presence within the virtual world. Input device 260 facilitates user interactions with the virtual environment, providing control over actions and resource manipulation inside the scene.

### 2.3. Networking and Other Components

In some examples, the customisable medical training system and the VR display systems are connected via a network, such as a local area network (LAN) or a wide-area network like the internet. This allows for communication between the customisable medical training system and the VR display systems, enabling the rendering of the generated or modified virtual environment to the training recipients.

The networking capabilities of the system may provide several advantages, such as allowing multiple users to participate in the same training scenario simultaneously, enabling collaboration and teamwork. Additionally, the network connection may allow supervising users to monitor and modify the virtual environment in real-time, providing valuable feedback and guidance to the training recipients.

In examples where a user acts as a supervising user outside the virtual environment (including but not limited to where user 130 supervises the training using system 110), the system used by the supervising user to monitor (and optionally further modify) the virtual environment and the training recipients' experience (the "trainer station" may be a standalone PC comprising an in-built router. Advantageously, such a configuration avoids issues associated with network traffic being unintendedly restricted by the firewall of the organisation in which system 100 is implemented, thus providing a system which can be set up and configured for use more quickly and easily, with delays eliminated.

Overall, the system and component details described in this section provide a comprehensive and flexible platform for customisable medical training, offering numerous advantages in terms of user experience, effectiveness, and adaptability to various training scenarios and requirements.

### 3. User Experience

The user experience will now be described for several aspects of the customisable medical training system, focusing on the generation and modification of the virtual environment via a graphical user interface (GUI) prior to rendering in virtual reality (VR), the training recipient experience, and the supervising user experience both inside and outside the virtual environment.

### 3.1. Generating/modifying a virtual environment via a GUI prior to rendering in VR

In one example, the customisable medical training system 110 may enable a customisation input to be provided via an interaction with a GUI 400 to allow users to generate or modify one or more virtual environments. GUI 400 may, for example, be displayed to a user 130 via a display 128, and the interaction may be facilitated via a user input device 126. One exemplary such interaction and GUI will now be described with reference to Figs. 4a-4c, which illustrate one possible workflow for entering customisation inputs to generate or modify a virtual environment. It will be appreciated that the following examples are provided for purposes of illustration rather than limitation, and that suitable modifications and variations will be readily apparent to those of ordinary skill in the art. The use of a GUI for customisation provides an easy and intuitive way for users to create and modify virtual environments tailored to specific training scenarios.

Referring first to Fig. 4a, there is illustrated a first aspect of GUI 400 that may be presented to a user at the commencement of a workflow, comprising a plurality of preexisting or pre-saved virtual environments 410 from which a selection may be made using the user input device 126. Preexisting virtual environments 410 may also be referred to as "template" virtual environments, since each such possible selection represents a preset format for a virtual environment (with its own virtual patient state, virtual medical resources, behaviours, and so forth) that serves as a model to be copied and further adapted. Data for each of the virtual environments 410 may be saved to a suitable location such as a database, and subsequently loaded when the time comes for any one specific virtual environment to be simulated to the users 155, 165, 175 (at which point, the relevant data can be downloaded or streamed to VR display devices 150, 160, 170 to provide said users with a VR experience). The location to which the data for the virtual environments 410 may be saved may be a separate location such as a separate database, or it may be part of (e.g., a database of) system 110, or part of (e.g., a database of) the host server.

GUI 400 further includes control means 430. Control means 430 may comprise means for deleting virtual environments 410, which can be advantageous if storage space limitations are exceeded. Control means 430 may comprise means for editing virtual environments, which can be advantageous if a new virtual environment is desired that shares a large number of elements in common with one of the preexisting virtual environments 410, or if one of the preexisting virtual environments 410 becomes outdated or obsolete and must be updated (e.g., due to a change in a hospital's processes, procedures or policies, or due to a change in availabilities or suitabilities of drugs, equipment or other resources). Control means 430 may comprise means for initiating a process of generating a new virtual environment from scratch, which may be useful if no suitable template is available.

Referring now to Fig. 4b, a second aspect of GUI 400 may be presented to a user following an interaction with either the "create new" or "edit" controls of the first aspect. GUI 400 include various input fields 420 and options for customising the state of the virtual patient 320 and the set of available virtual medical resources 330. In the case of a user providing a customisation input to create a brand-new virtual environment, all input fields 420 may be initially blank in the second aspect of GUI 400 (and/or set to default values). In the case of a user providing a customisation input to edit or modify an existing virtual environment (such as a template virtual environment), fields 420 may comprise one or more custom values previously selected by a user (not necessarily the same user).

Fields 420 may include, but are not limited to: text fields (single-line or multi-line), numeric fields, drop-down menus, radio buttons, checkboxes, sliders, file upload fields, search fields, date/time pickers or calendars, and the like. Some of fields 420 may include autocomplete fields configured to predict and suggest options based on partial user input. Some of fields 420 may optionally be configured to accept multiple entries in the same field. For example, a field for input of a "lower limbs pathology" may enable the input of both a "fibular fracture" pathology and a "tibial fracture" pathology in the same field.

Once the user has finished filling in fields 420 to their satisfaction, they may progress to the next aspect of GUI 400 using user input device 126, for example by pressing the "enter" key on a keyboard or by using a mouse to click on suitable control means on GUI 400 for advancing. Alternatively, a user may cancel their customisation input using user input device 126, for example by pressing the "escape" key on a keyboard or by using a mouse to click on suitable control means on GUI 400 for exiting GUI 400 (or at least the second aspect thereof). The user may be permitted to advance to the next (i.e., third) aspect of GUI 400 even if not all of fields 420 have been completed. For example, some fields may be optional, or may be configured to take a predetermined "default" value if a user does not explicitly complete them. Alternatively, even if all of the fields 420 are mandatory and cannot be (or are not) assigned any default value, the user may nevertheless be permitted to advance to the third aspect of GUI 400 and even to save their work in progress, subject to the provision that it will not be possible to launch the simulation of their virtual environment until the user (or another user) returns to finish populating all of the mandatory fields 420 in GUI 400.

Referring now to Fig. 4c, a third aspect of GUI 400 may be presented to a user once the user is satisfied with their entries to the fields 420. This aspect provides a "summary" or "confirmation" view of the user's customisation input for the virtual environment, summarising some or all of said input in a concise read-only format for the user to check (e.g., for errors). When the user has checked and is satisfied with the details for the generated/modified virtual environment, they may interact with control means 430 to save the virtual environment or a copy thereof. Control means 430 can optionally include means for overwriting an existing template virtual environment based on the new customisation input, and/or means for using the new customisation input to create a brand new virtual environment without overwriting the template currently being worked on. If the user is not satisfied that the specified values are correct, they may return to the second aspect of GUI 400 using user input device 126, for example by pressing the "escape" key on a keyboard or by using a mouse to click on suitable control means on GUI 400.

### 3.2. Training recipient experience

The experience as perceived by a training recipient will now be described with reference to Fig. 6. As explained above, a training recipient 155, 175 may use a VR display system 150, 170 to interact with the virtual environment 300. The VR display system may comprise a head-mounted device (HMD) 200 with a display 240, an audio device 250, and motion sensors 270. The training recipient may also use one or more input devices 260, such as handheld controllers, to interact with the virtual environment and manipulate virtual medical resources 330. This immersive and interactive experience allows the training recipient to engage in hands-on training in a realistic and engaging virtual environment substantially as follows.

The training recipient views a 3D virtual scene using the HMD of their VR display system, the 3D virtual scene comprising meshes and/or surface geometry, shading, textures and the like, in a configuration designed to replicate at least partially a real medical or surgical environment. For example, the scene may be a virtual room in a hospital or other medical facility (e.g., a surgical theatre), the interior of an ambulance, or a roadside site of a traffic collision. The training recipient may be able to look around inside the scene (modifying the pitch, yaw and/or roll angle of their field of view) via head movements detected by the motion sensor of the HMD as described hereinabove. The training recipient may be able to move around inside the scene (modifying at least the horizontal position coordinates of their field of view, and optionally the vertical position coordinate) via head movements detected by the motion sensor of the HMD as described hereinabove. Since the amount of real-world space available to a training recipient in e.g., a bedroom or office may in various cases be substantially less than the size of the 3D virtual scene, the training recipient may be able to use input device(s) 260 to move greater distances around the virtual scene, for example using gesture-based controls, than would be possible by only using motion sensor 270 to track the movement of the training recipient's head in the real world. In one example, the VR display system permits the user to indicate a desired target position on the ground within the virtual scene via hand tracking, by making a "pointing" motion with their index finger towards said target position and then curling the index finger back towards the hand into a bent position to effect the movement. This can serve essentially to "teleport" the user to the desired position. Of course, a variety of other suitable means for movement within the 3D virtual scene will be readily apparent to those of ordinary skill in the art.

The training recipient also views any other training recipients and/or supervising users who are present in the virtual environment or virtual scene (i.e., who are simultaneously themselves viewing and interacting with the same virtual environment). As seen in Fig. 6, the training recipient's view comprises another training recipient. This serves to replicate the format of many existing simulation centres, in which training recipients collaboratively work in the same physical location on the same virtual patient. In this way, the training recipients develop situational awareness of their surroundings and of the actions/behaviours of the other members of their team. In one example of the invention, one or more of VR display systems 150, 160, 170 can comprise microphones to permit communication in the form of speech between training recipients and supervisors (and/or other training recipients) across the network 130 using respective audio devices 250.

Additionally or alternatively, training recipients and/or supervisors may be able to mutually communicate via other suitable means such as various forms of real-time text-based messaging. For instance, in some examples, a training recipient may be assigned a "scribe"-type role in which at least one of their objectives during the session is to document a record of events that occur. This "scribe" training recipient may be a training recipient "outside" of the VR environment, in the sense that they may be viewing the VR environment through non-VR means (in the same way that a supervising user 130 may be external to the VR environment whilst still monitoring and/or managing said environment via non-VR means). The scribe may use suitable input means (e.g., a keyboard) to enter information about various critical steps and events occurring (e.g., drugs administered, blood given, vital signs) whilst the other training recipient(s) complete the virtual procedure. Information entered by the scribe may be communicated to the other training recipient(s) in real time, for example by appearing directly on their GUI(s), or by appearing within one or more documents accessible by the other training recipients via GUI elements or via interaction with the virtual environment. In some examples, the other (non-scribe) training recipient(s) may be unable to respond to messaging information arising from the scribe's input - advantageously, this removes one possible source of distraction and permits them to focus on completion of the virtual procedure.

In some examples, a supervising user or trainer outside of the virtual environment (e.g., a user of the "trainer station", who may view an interface similar to that shown in Fig. 7) may communicate with one or all of the training recipients by entering textual information. The textual information may be text documenting one or more events occurring in the virtual environment; such events may include for example patient events, actions performed by the training recipient(s), actions performed by the supervising user, and so forth. The textual information may be displayed either within the virtual environment (e.g., on a virtual display screen or other virtual medical resource), or otherwise on the user or users' VR display device(s) (e.g., as a 2D overlay). Advantageously, training recipients can use this text, input by the supervisor/trainer acting effectively as a "scribe", as a reference to assist with documenting the ongoing virtual procedure.

A virtual patient 320 is present inside the virtual scene, replicating the manikin which would typically be present in a traditional simulation centre as known in the art. Advantageously, because the behaviour of virtual patient 320 is not produced by physical means, a much wider range of simulated behaviours and greater extent of customisability can be provided in the virtual patient compared to these manikins. The training recipient may be able to view, move and interact with the virtual patient. Movement of the virtual patient may comprise rotation (pitch/yaw/roll) of the virtual patient relative to the virtual scene. Interaction with the virtual patient may comprise, at the most basic level, direct interaction by the training recipient with the body of the virtual patient itself, e.g., by means of tracked hand motions. In many cases, however, the training recipient will interact with the virtual patient using one or more virtual medical resources as described hereinbelow.

The training recipient may see and interact with a variety of virtual medical resources displayable in the 3D virtual scene. By "displayable", what is meant is that at any given time there may be a first set of virtual medical resources that appear in the 3D virtual scene and are visibly rendered to the training recipient(s), as well as a second set of virtual medical resources which are not present within the 3D virtual scene in that instant, but which nevertheless belong to the virtual environment and are ready for displaying (i.e., capable of being displayed) in the 3D virtual scene. A displayable virtual medical resource which is not currently displayed in the 3D virtual scene may be displayed upon a particular action or interaction by the training recipient.

For example, the virtual environment may comprise a displayable virtual medical resource in the form of a blood bag, and may be configured such that the training recipient is able to interact with a virtual medical resource or part of the 3D virtual scene in the form of a cupboard or cabinet to cause the blood bag to be displayed in the 3D virtual scene. As another example, the virtual environment may comprise a resource in the form of a drug or pharmaceutical product, and may be configured such that the training recipient can interact with a virtual medical resource or part of the 3D virtual scene in the form of a drawer or bag to cause the drug or pharmaceutical product to be displayed in the 3D virtual scene. As another example, the virtual environment may comprise a virtual medical resource in the form of a 2D image asset such as an X-ray, ultrasound scan, CT image, or sequence thereof. The virtual environment may be configured such that the training recipient can interact with a virtual medical resource or part of the 3D virtual scene in the form of a virtual display device, scanning or imaging apparatus, or other controls, to cause the 2D image asset to be displayed in the 3D virtual scene. As another example, the virtual environment may comprise a virtual medical resource in the form of a text asset, such as one or more virtual results of laboratory tests, and may be configured such that the training recipient is able to interact with a virtual medical resource or part of the 3D virtual scene in the form of a virtual printout, clipboard, document, book, screen, tablet, or other device, to cause the text asset to be displayed in the 3D virtual scene.

In this way, the action/interaction of the training recipient can cause a displayable virtual medical resource effectively to move from the first "displayed" set to the second "non-displayed" set, or vice versa.

One or more virtual medical resources may be configured to appear in a static or fixed position within the 3D virtual scene. One or more virtual medical resources may be moveable via interaction by the training recipient and/or supervising user. A 3D virtual scene may simultaneously comprise both fixed and moveable virtual medical resources. Moveable virtual medical resources may optionally (but by no means need necessarily) comprise virtual medical resources which are subject to physics effects (e.g., configured to fall to the ground if dropped, to slide or roll on inclined surfaces, to detect and react to collisions with other virtual medical resources, and so forth). Moveable virtual medical resources may be configured to be "picked up" and held in the (virtual) hand of the training recipient, with this behaviour being triggered upon the training recipient moving their hand or controller to a position corresponding to that of the virtual medical resource and performing an appropriate action, such as pressing a button or trigger (in the case where their input device is a controller) or making an appropriate gesture, such as a grasping or grabbing gesture (in the case of hand tracking). Virtual medical resources comprising virtual keys or buttons (whether these are present to simulate analogous equipment in the real world or simply for ease of interaction) may be configured for interaction by the training recipient such that the training recipient can use their controller or hand tracking to press said keys or buttons inside the virtual environment (e.g., in the latter case, with a straight extended finger). Visual feedback may be displayed - on a virtual medical item/tool, a virtual display, a virtual consumable medical/pharmaceutical product or any other appropriate virtual medical resource - based on an outcome of a virtual procedure or of a step thereof.

The virtual patient may be configured to enable a variety of ways of using the virtual medical resources. In one example, the virtual patient is configured to visibly undergo pupil dilation when the training recipient "picks up" a surgical light source (such as a torch) as described above and manoeuvres it by means of their input device to a position adjacent or proximate to an eye of the virtual patient. IV lines or ECG leads may be introduced by moving them to a suitable position on the body of the virtual patient. Surgical instruments such as thermometers may be picked up and used on an appropriate location of the virtual patient to obtain a readout useable by the training recipient to inform surgical decision-making of their team. In some cases, more complex behaviours and interactivity can be provided for a virtual medical resource. For instance, in the case where the virtual medical resource is a syringe comprising a drug, the training recipient may interact with the resource by making a first gesture (e.g., pressing a button of a controller or pressing a virtual button in the virtual environment appearing within their field of view) to specify a dosage for the drug, then move nearer to the virtual patient, and finally inject the virtual patient with the drug by making a second gesture (e.g., pulling a trigger on a controller or closing their hand in a tracked gesture).

In one example, each training recipient may be provided in the virtual environment with access to a personal virtual control specific and available only to them (and not to other training recipients or the supervising user). In a preferred implementation, this personal virtual control takes the form of a virtual "smart watch" located on the training recipient's virtual wrist - that is, appearing in the training recipient's field of view whenever their head position and hand position are aligned such that the training recipient would see their wrist if they were not wearing the head-mounted device, and appearing at the position in said field of view corresponding to the same position on their wrist in the real world. The personal virtual control may be interacted with via gesture, motion or other suitable controls to interact with the virtual patient and/or various virtual medical resources to perform various actions (e.g., viewing imaging data on their own display, or causing said imaging data to be displayed on one or more virtual display screens present in the virtual environment).

In some optional (though non-essential) examples, where hand tracking is used to enable interaction between a training recipient and the virtual patient, simulated physics may be provided in the virtual environment to detect an external geometry or mesh of the virtual patient and prevent a hand of the training recipient from intersecting (e.g., cutting through) said geometry/mesh in the virtual environment. That is, wherever the training recipient may position their hand(s) in the real world, their simulated VR "hands" will not intersect the virtual patient, thus breaking the disbelief and realism of the virtual environment.

By interacting with the virtual patient in the various ways disclosed herein (including via use of one or more virtual medical resources, for example by administering virtual pharmaceutical products to the virtual patient), the one or more training recipients may be enabled to change the state of the patient in real time within the virtual environment. For one example, the virtual patient's respiratory rate may be changed by the training recipient interacting with the virtual patient e.g., using a virtual bag-valve-mask (BVM) resource. As another example, by administering rocuronium to the virtual patient, a training recipient may affect the Clinical Global Impression (CGI) value of the patient, particularly in the CGI-Severity (CGI-S) component. Additionally, or alternatively, this action may affect the virtual patient's state by changing the virtual patient's external appearance, animation state, and so forth. This may have the effect of overwriting or overriding a customised CGI/CGI-S value (or external appearance/animation state) that has been previously set using a customisation input. A virtual monitor may be provided to display vital signs of the virtual patient (blood pressure, respiratory rate, and so forth) within the virtual environment. The actions of the training recipients within the virtual environment may directly affect these values, for example using a BVM resource when the patient is not voluntarily breathing, as explained above. In some situations, the values may be set manually by a customisation input from a supervising user (either within or outside the virtual environment) - for example, after intubation, a training recipient checks the virtual monitor to confirm success by watching for a rise in the virtual patient's SpOz value (which is set manually in real time by a supervising user).

### 3.3. Supervising user inside the virtual environment

In some examples, a supervising user 165 may also use a VR display system 160 to enter and interact with the virtual environment 300. The supervising user may in general experience the virtual environment in the same way that any other user (e.g., training recipient 155, 175) does, but may be provided by virtue of their role as a trainer or supervisor with access to additional or enhanced functionality within the virtual environment, such as the ability to provide a customisation input to modify the virtual environment, e.g., in real time. That is, the act of communicating with VR display 160 to render a virtual environment to supervising user 165 may comprise substantially the same steps and operations as communicating with VR display 150 to render the virtual environment to training recipient 155, but with supervising user granted additional freedoms and controls to manipulate the virtual environment such as (but not limited to) the ability to interact with at least a component of VR display 160 to provide a customisation input to modify the virtual environment (or generate a new one).

For example, referring now to Fig. 5, a graphical user interface 500 as seen by the supervising user may be substantially the same as that seen by a training recipient, and may comprise a personal virtual control 520 (optionally in the form of a "smart watch") as described hereinabove. Interaction with the personal virtual control can cause a menu 540 to be displayed within GUI 500 to the supervising user, the menu comprising one or more interactable elements for performing actions inside (and/or changing the state of) the virtual environment or 3D virtual scene. The interactable elements may be configured upon initiation of an interaction by the supervising user to detect, display or alter the state of the virtual patient, and/or to provide access to a virtual medical resource for detecting, displaying or altering the state of the virtual patient, for example.

Among the one or more interactable elements there may be included one or more first "common" interactable elements 560 configured to be displayed to and accessible by any one of the training recipients upon interaction with the personal virtual control. Additionally or alternatively, among the one or more interactable elements there may be included one or more second "restricted" or "supervisor-only" interactable elements 580 configured to be displayed to the supervising user only (upon interaction with their personal virtual control), or configured to be displayed to all users but accessible/useable by the supervising user only (upon interaction with the person virtual control). In the latter case, interactable elements 580 may e.g., appear greyed-out to training recipients (and anyone else present in the virtual environment not having the privileges of the supervising user) to provide a visual indication that no interaction is available to them.

Examples of interactable elements 580 and their functionalities may include elements for modifying the virtual patient state (e.g., for modifying their vital signs), elements for adding/removing virtual medical resources to/from the 3D virtual scene, elements for triggering or modifying playback of digital audio assets, and/or elements for modifying general settings, parameters and characteristics of the virtual environment as a whole. In some examples, elements for modifying the virtual patient state may comprise elements for opening or closing the eyes of the virtual patient, elements for moving the limbs of the virtual patient, and so forth. One or more elements of the interface shown graphically to the supervising user (e.g., virtual patient vital signs, alerts/warnings, virtual medical resource details, notifications, and so forth) may be updated in response to actions taken by the supervising user or by other users within the virtual environment.

The enhanced experience and functionality provided to the supervising user allows the supervising user to actively monitor and adjust the training scenario in real-time, providing a more dynamic and responsive training experience for the training recipient.

### 3.4. Supervising user outside the virtual environment

In some examples, a supervising user (not necessarily the same supervising user mentioned above) may monitor and modify the virtual environment from outside the virtual environment via their own display means and user interface. This "external" supervising user may optionally provide customisation input via the GUI to generate or modify the virtual environment, which is then rendered to the training recipient via the VR display system. Data may optionally be presented to the supervising user via the GUI, representative of the state of the virtual environment and elements thereof (e.g., the virtual patient state). The presented data may be data which is in some way "hidden" from the training recipients, at least in the sense of the recipients not being always directly aware of the data. For example, a patient's heart rate and respiratory rate may not be known to the training recipients without the use of virtual medical resources to measure and/or count breaths and beats, but both of these values may be presented to the supervising user at all times (or at any time of their choosing). When the training recipient(s) take actions in the virtual environment affecting the patient state (in the above example, this may be e.g., using a bag-valve-mask device (BVM) to deliver oxygen ventilation), the data presented to the supervising user can be updated. All of this allows the supervising user to have a broader view of the training scenario and make adjustments as needed without being immersed in the virtual environment themselves.

Referring now to Fig. 7, there is depicted a typical view which may be available to an external supervising user who is outside the virtual environment (i.e., who is not using a VR display device to interact with the virtual environment). The external supervisor's view may comprise: one or more views of the 3D virtual scene and resources therein as seen by one or more of the in-VR users (i.e., from the perspective(s) of the training recipients and/or supervising users that are currently immersed in the virtual environment); one or more objective views of the 3D virtual scene (either a view from a fixed position and angle or a moveable view which can be adjusted to cover different parts of the scene and different angles) that are not specific to any user; one or more presentations of the global state of the virtual environment (including the virtual patient state, user states, virtual medical resource states, digital audio asset states, and/or other relevant state information useful for monitoring and oversight of the ongoing simulation); and/or one or more controls for entering a customisation input to cause modification (e.g., real time modification) of the virtual environment.

In this way, it is possible to replicate and build upon traditional processes for oversight and assessment that are currently used in the art in relation to simulation centres.

The supervising user outside of the virtual environment may in some examples be user 130, i.e., the creator/customiser of the virtual environment, and the same entity responsible for providing the customisation input to do so. User 130 may be presented one or more views of the 3D virtual scene via display 128 and may case modification of the virtual environment via user input device 126. Additionally or alternatively, the supervising user outside of the virtual environment may be user 165, or may be some other (unpictured) entity responsible for supervision of the training. It is advantageous to have at least one supervising user outside the virtual environment, since such a supervising user can be afforded greater oversight, awareness and coverage of how the training recipients are performing, and moreover will be less distracted by the immersive VR experience itself, leaving them to focus on the training recipients rather than the difficulties and challenges inherent in navigating the virtual environment.

The supervising user outside of the virtual environment may in some examples be provided with access to a log via the GUI. This may comprise providing the supervising user with read access and/or write access to the log. The log may comprise a chronological collection of events, actions or messages generated by the server, the software, the training recipient(s) and/or the in-VR supervising user(s). The log may comprise, for each logged event, one or more of: event descriptions, timestamps, importance levels, and error codes. Read access to the log may provide the supervising user with capabilities such as one or more of: aggregation, searching, filtering, and visualisation (of the logged events). Advantageously, this can assist the supervising user in evaluating proficiency of the training recipients and enable them to provide constructive feedback during a post-session review.

The supervising user outside of the virtual environment may in some examples be provided via the GUI with means for managing voice communication among the training recipient(s) and supervising user(s) inside the virtual environment. For instance, the external supervising user may be provided with means for selectively muting (and/or adjusting the volume of) the voices of the training recipients. Advantageously, this can help to prevent the training recipients from becoming distracted by the voices of other training recipients in the VR environment when attention is required, for instance when the in-VR supervising user is speaking. In one example, a supervising user/trainer can thus be enabled to speak privately to one or more training recipients, such that voice communication from the supervisor to the training recipient (and/or vice versa) is not audible to the other training recipient(s). Advantageously, this allows feedback to be given in a manner preserving the privacy of the training recipient and without distracting the other training recipients.

In one example, the supervising user may be provided with a "voice grouping" feature whereby one or more groups of users are identified (training recipients, supervising users, or mixtures thereof) and muting is performed selectively such that utterances and other sounds produced by members of a group are not played to the group's other members (e.g., are not played by the VR display devices of the other group members). For example, a simulated surgical procedure may involve a first supervising user and four training recipients all interacting with a virtual environment simultaneously, using VR display devices. A second supervising user who is outside of the virtual environment may group the first supervising user together with two of the training recipients. Consequently, speech may be relayed (via suitable microphone, networking and audio playback means) from members of the group to the other two training recipients, from the other two training recipients to the members of the group, and bilaterally between the other two training recipients, but not between the members of the group. This feature advantageously improves training in the case where some of the system users occupy the same real-world space (e.g., the same room) as each other, since these users can already hear each other's voices in the real world and therefore benefit from having the redundant in-VR voice communication removed, eliminating a source of distraction. Multiple groups may be designated when training occurs across more than one site/space/room. Users who are not assigned a group may be considered to occupy their own group of size 1 for the purpose of determining how voice communication is relayed.

In summary, the customisable medical training system provides a flexible and immersive user experience for both training recipients and supervising users. The use of a GUI for generating and modifying virtual environments, combined with the immersive and interactive VR display system, allows for tailored and engaging training scenarios. The ability for supervising users to monitor and adjust the virtual environment both inside and outside the virtual environment provides a dynamic and responsive training experience for the training recipient.

### 4. Customisable Details

With reference made now to Fig. 3, there is provided a detailed description of various customisable details of the virtual environment in the customisable medical training system. These customisable details allow for a tailored and immersive training experience for the training recipient.

### 4.1. Virtual Patient State

In one example, the state of the virtual patient can be customised to create a realistic and engaging training scenario. The virtual patient state may comprise various attributes, such as manikin type and appearance, observations on arrival, signs, airway, breathing, circulation, disability, and pathology.

### 4.1.1. Manikin Type and Appearance

In some examples, the virtual patient's appearance can be customised based on factors such as age, gender, ethnicity, position, and clothing state. This allows for a diverse range of virtual patient scenarios to be simulated, enabling training recipients to practice their skills on a variety of patient types.

### 4.1.2. Observations on Arrival & Vital Signs

In one example, the virtual patient's vital signs can be customised, including body temperature, heart rate, respiration rate, blood pressure, oxygen saturation, blood glucose level, blood CO₂ level (or indeed, any suitable blood property determinable by testing), and end-tidal CO₂ level. This allows for the creation of tailored training scenarios that focus on specific medical conditions or emergencies.

### 4.1.3. Airway

In some examples, the virtual patient's airway can be customised, including factors such as airway patency, airway obstruction, and the presence of foreign bodies. This enables training recipients to practice airway management techniques in various scenarios.

### 4.1.4. Breathing

In one example, the virtual patient's breathing can be customised, including factors such as respiratory rate, respiratory effort, and the presence of abnormal breath sounds. This allows for the creation of training scenarios that focus on respiratory emergencies and interventions.

### 4.1.5. Circulation

In some examples, the virtual patient's circulation can be customised, including factors such as blood pressure, heart rate, and the presence of abnormal heart sounds. This enables training recipients to practice circulatory assessment and management techniques in various scenarios.

### 4.1.6. Disability

In one example, the virtual patient's disability can be customised, including factors such as level of consciousness, pupil size, pupil reactivity, and motor function. This allows for the creation of training scenarios that focus on neurological emergencies and interventions.

### 4.1.7. Pathology

In some examples, the virtual patient's pathology can be customised, including factors such as skeletal pathology, respiratory pathology, and circulatory pathology. This enables training recipients to practice assessment and management techniques for various medical conditions and emergencies.

### 4.2. Virtual Medical Resources and Behaviour

In one example, the virtual environment may comprise a set of available virtual medical resources that can be displayed and interacted with in the 3D virtual scene. These resources may include items and consumables, tools and equipment, pharmaceuticals, virtual displays, 2D media assets and text assets. Each virtual medical resource may optionally be associated with one or more virtual resource behaviours, allowing for realistic interactions between the training recipient and the virtual resources.

In some optional examples, various virtual medical resources (including but not limited to items/consumables, tools/equipment, and pharmaceuticals) may be associated with a first, "success", behaviour and a second, "failure" behaviour. Rendering the virtual environment may comprise: in response to a training recipient using a virtual medical resource to interact with the virtual patient, determining an outcome status of the interaction; in response to the determined outcome status being positive, rendering the virtual medical resource set to its first behaviour; or, in response to the determined outcome status being negative, rendering the virtual medical resource set to its second behaviour. The first/second behaviour may comprise providing respective first/second feedback (e.g., visual feedback or audio feedback) to the training recipient that used the virtual medical resource. For example, use of a virtual intravenous drip may result in a success behaviour or failure behaviour by the virtual intravenous drip. Advantageously, the display/rendering of failure-type behaviours may encourage the training recipient to re-examine the virtual patient, or take an alternative step to complete the procedure.

In some optional examples, various virtual medical resources (including but not limited to items/consumables, tools/equipment, and pharmaceuticals) may provide different behaviours depending on the state of the virtual patient on which they are used. For example, the output of a chest tube drainage canister may vary depending on the pathology state of the virtual patient. As another example, the behaviour of a laryngoscope (e.g., the output images that are displayed to the training recipient) may vary depending on the otolaryngological pathology (e.g., throat contents) of the virtual patient.

In some optional examples, various virtual medical resources may be set to provide different behaviours based on the customisation input, irrespective of the state of the virtual patient on which they are used. The designer of a training scenario may desire a particular chosen behaviour to be displayed by a virtual medical resource regardless of the patient's state. For example, in order to test training recipients' ability to improvise, adapt, overcome and respond to setbacks encountered in the course of a virtual medical procedure, a customisation input may be provided to cause a particular virtual medical resource (e.g., item, tool, piece of equipment or the like) to simulate a "failure" behaviour when any training recipient attempts to use the virtual medical resource on the virtual patient, whatever the virtual patient state. This may comprise setting e.g., a virtual IV line or drainage cannister to have a predetermined behaviour set when the virtual environment is generated or modified. In this way, virtual medical resource behaviours can be pre-programmed without having to depend at all on the patient state, with the customising user having full control to decide on the feedback given by virtual medical resources at the time that the customisation is performed (either before or during the virtual medical procedure), based on learning outcomes.

### 4.2.1. Items and Consumables

In some examples, the virtual environment may comprise a variety of virtual medical items and consumables, such as bandages, dressings, syringes, and intravenous fluids. These items can be used by the training recipient to practice various medical procedures and interventions.

### 4.2.2. Tools and Equipment

In one example, the virtual environment may comprise a variety of virtual medical tools and equipment, such as stethoscopes, blood pressure cuffs, and defibrillators. These tools can be used by the training recipient to practice assessment and management techniques in various scenarios.

### 4.2.3. Pharmaceuticals

In some examples, the virtual environment may comprise a variety of virtual pharmaceutical products, such as medications, intravenous fluids, and oxygen. These pharmaceuticals can be used by the training recipient to practice medication administration and management techniques in various scenarios.

### 4.2.4. Virtual Displays

In one example, the virtual environment may comprise a variety of virtual displays, such as monitors, screens, and charts. These displays can be used by the training recipient to access and interpret virtual patient information, such as vital signs, medical history, and laboratory results. Other forms that a virtual display may take include virtual printouts, virtual clipboards, virtual documents, virtual books, and the like.

### 4.2.5. 2D Media Assets

In some examples, the virtual environment may comprise a variety of 2D media assets, such as images, videos, and documents. These assets can be displayed on virtual displays within the 3D virtual scene, allowing the training recipient to access and interact with relevant information during the training scenario.

### 4.2.6. Text Assets

In some examples, the virtual environment may comprise a variety of text assets, such as one or more virtual results of laboratory tests, virtual patient records text, and/or simulated text received from a first responder. These assets can be displayed on virtual displays or other resources within the 3D virtual scene for the benefit of the training recipient.

### 4.3. Adding/Removing Resources to/from the Scene

In one example, the training recipient or supervising user may interact with the virtual environment to add or remove virtual medical resources from the 3D virtual scene. This allows for a dynamic and adaptable training experience, enabling the training recipient to practice with a variety of resources as needed. In some examples, a customisation input can be provided to add/remove virtual medical resources individually (or a few at a time). In some examples, a customisation input can be provided to add/remove entire sets of displayed or displayable virtual medical resources by changing the environment that is being simulated. For example, based on the requirements of the training, a user may provide a customisation input to cause the virtual environment to simulate e.g., a hospital ward, a surgical theatre, a roadside accident site, the interior of an ambulance, a residential building, and so forth. This may comprise adding/removing a plurality of virtual medical resources to the set of displayable virtual medical resources, adding/removing a plurality of virtual medical resources to the set of displayed virtual medical resources, and/or changing the 3D virtual scene.

### 4.4. Digital Audio Assets

In some examples, the virtual environment may comprise a set of digital audio assets, such as sound effects, voice recordings, and background noise. These audio assets can be played in the virtual environment to the training recipient(s) to enhance the realism and immersion of the training experience. Rendering the virtual environment may comprise playing one or more audio assets continually (e.g., as a loop), to provide background or ambient sound. Rendering the virtual environment may comprise playing one or more audio assets based on the state of the virtual patient. For example, an alert sound may be played corresponding to a state of the virtual patient, such as playing an alert sound when one or more simulated vital signs go outside of an identified threshold range (i.e., a "safe" window). One or more audio assets may be audible/played only when certain actions are taken by the training recipient, e.g., when the recipient is within a specific range of the virtual patient, or when the recipient uses a particular virtual medical resource on the virtual patient (e.g., a stethoscope). One or more audio assets may be played in response to a state of the virtual patient being changed, which may occur due to actions taken by the training recipients and/or the supervising user.

The digital audio assets may comprise, inter alia, body sounds, organ sounds, breathing sounds, heartbeat sounds, abdomen sounds, palpitation sounds, alert sounds, warning sounds, and the like.

### 5. Miscellaneous

The customisable medical training system may be designed to facilitate collaboration between multiple training recipients and supervising users. This collaboration can occur in various ways, such as simultaneous use by multiple training recipients, supervision from within the virtual environment, and supervision from outside the virtual environment.

In some examples, the customisable medical training system may allow multiple training recipients to simultaneously interact with the virtual environment. This can be achieved by rendering the generated or modified virtual environment to each training recipient via their respective VR display systems. The training recipients can then collaborate in real-time within the virtual environment, allowing them to practice teamwork and communication skills in addition to their individual medical skills. This feature provides an advantage by enabling more realistic and effective training scenarios that mimic real-life medical situations where teamwork and communication are crucial.

In some examples, the customisable medical training system may allow one or more supervising users to enter the virtual environment alongside the training recipients. The supervising users can observe the training recipients' actions and provide guidance, feedback, or assistance as needed. This feature allows for more effective supervision and a more immersive learning experience for the training recipients.

In some examples, the customisable medical training system may allow multiple supervising users to enter the virtual environment simultaneously. This can be particularly useful in complex training scenarios where multiple areas of expertise are required. Each supervising user can focus on specific aspects of the training, providing more targeted feedback and guidance to the training recipients. This feature enhances the overall effectiveness of the training experience by ensuring that all aspects of the training scenario are adequately supervised and addressed.

In some examples, the customisable medical training system may allow one or more supervising users to observe and interact with the virtual environment from outside the virtual environment. This can be achieved by providing the supervising user with a graphical user interface that displays a view of the virtual environment and allows the supervising user to provide customisation input to modify the virtual environment in real-time. This feature enables supervising users to monitor and adjust the training scenario without being directly immersed in the virtual environment, allowing for more efficient supervision and the ability to manage multiple training scenarios simultaneously.

In some examples, the customisable medical training system may allow multiple supervising users to observe and interact with the virtual environment from outside the virtual environment simultaneously. Each supervising user can provide customisation input to modify the virtual environment independently, allowing for more targeted and efficient supervision. This feature enables multiple supervising users to collaborate in managing and adjusting the training scenario, ensuring that all aspects of the training are adequately supervised and addressed.

The customisable medical training system may support various combinations of simultaneous use by multiple training recipients and supervision by multiple supervising users, both within and outside the virtual environment. For example, the system may allow two training recipients to collaborate within the virtual environment while being supervised by one supervising user within the virtual environment and another supervising user outside the virtual environment. This flexibility in collaboration and supervision options enables the customisable medical training system to accommodate a wide range of training scenarios and supervision requirements, providing a highly adaptable and effective medical training solution.

In some examples, the customisable medical training system may be configured to integrate with external systems and databases, such as scan data file databases, electronic health record systems, medical databases, or other sources of patient information. This integration can allow for the automatic generation or modification of virtual medical resources and/or virtual patient states based on real-world patient data, providing a more realistic and relevant training experience.

In some examples, the customisable medical training system may comprise performance tracking and assessment features, allowing for the monitoring and evaluation of a training recipient's performance during a training session. This can include tracking the user's interactions with the virtual patient and virtual medical resources, as well as measuring the user's response times, accuracy, and adherence to medical protocols. The system may generate performance reports or provide real-time feedback to the training recipient or supervising user, facilitating continuous improvement and skill development.

In some examples, the customisable medical training system may comprise a scenario library, allowing users to save, share, and access pre-built or custom training scenarios. This can facilitate collaboration between users and enable the efficient reuse of training scenarios across multiple training sessions or institutions. The scenario library may be stored locally or in a cloud-based storage system, allowing for easy access and sharing among users.

In some examples, the customisable medical training system may be designed to support multiple platforms, such as desktop computers, laptops, tablets, or smartphones, in addition to VR display systems. This can provide users with greater flexibility in accessing and interacting with the system, as well as enabling remote supervision or collaboration between users in different locations.

The term "comprising" encompasses "including" as well as "consisting" e.g. a composition "comprising" X may consist exclusively of X or may include something additional e.g. X + Y.

Unless otherwise indicated each example or implementation as described herein may be combined with another example or implementation as described herein.

The methods described herein may be performed by software in machine readable form on a tangible storage medium e.g. in the form of a computer program comprising computer program code means adapted to perform all the steps of any of the methods described herein when the program is run on a computer and where the computer program may be embodied on a computer readable medium. Examples of tangible (or non-transitory) storage media include disks, hard-drives, thumb drives, memory cards, etc. and do not include propagated signals. The software can be suitable for execution on a parallel processor or a serial processor such that the method steps may be carried out in any suitable order, or simultaneously. This acknowledges that firmware and software can be valuable, separately tradable commodities. It is intended to encompass software, which runs on or controls "dumb" or standard hardware, to carry out the desired functions. It is also intended to encompass software which "describes" or defines the configuration of hardware, such as HDL (hardware description language) software, as is used for designing silicon chips, or for configuring universal programmable chips, to carry out desired functions.

Those skilled in the art will realise that storage devices utilised to store program instructions can be distributed across a network. For example, a remote computer may store an example of the process described as software. A local or terminal computer may access the remote computer and download a part or all of the software to run the program. Alternatively, the local computer may download pieces of the software as needed, or execute some software instructions at the local terminal and some at the remote computer (or computer network). Those skilled in the art will also realise that by utilizing conventional techniques known to those skilled in the art that all, or a portion of the software instructions may be carried out by a dedicated circuit, such as a DSP (Digital Signal Processor), programmable logic array, or the like.

It will be understood that the benefits and advantages described above may relate to one example or may relate to several examples. The examples are not limited to those that solve any or all of the stated problems or those that have any or all of the stated benefits and advantages.

The steps of the methods described herein may be carried out in any suitable order, or simultaneously where appropriate. Additionally, individual steps may be deleted from any of the methods without departing from the scope of the subject matter described herein. Aspects of any of the examples described above may be combined with aspects of any of the other examples described to form further examples without losing the effect sought. Any of the steps or processes described above may be implemented in hardware or software.

It will be understood that the above descriptions of preferred examples, instances and implementations are given by way of example only and that various modifications may be made by those skilled in the art. Although various examples have been described above with a certain degree of particularity, or with reference to one or more individual examples, those skilled in the art could make numerous alterations to the disclosed examples without departing from the scope of this invention (which is defined by the appended claims).

In conclusion, the customisable medical training system described herein provides a highly flexible, immersive, and realistic training experience for medical professionals. The system's ability to generate or modify virtual environments based on customisation input, combined with its support for a wide range of virtual patient states, virtual medical resources, and additional features, enables users to tailor their training scenarios to their specific needs and objectives. This can lead to more effective and efficient medical training, ultimately improving patient outcomes and the overall quality of healthcare.

The following is a list of numbered embodiments which further serve to aid understanding of the invention, and which may or may not be claimed:
1. A customisable medical training system comprising a processor and a memory, the memory containing instructions which, when executed by the processor, cause the processor to:
   generate or modify, based on a customisation input, a virtual environment comprising:
      a 3D virtual scene in which a virtual patient is displayed,
      a state of the virtual patient; and
      a set of one or more available virtual medical resources displayable in the 3D virtual scene; and
   communicate with a VR display system to render the generated or modified virtual environment to a training recipient via said VR display system;
   wherein the customisation input is a manual input received to customise the state of the virtual patient and/or the set of available virtual medical resources.
2. The system of embodiment 1, wherein generating or modifying the virtual environment comprises modifying an existing virtual environment rendered to the training recipient.
3. The system of embodiment 2, wherein the VR display system is a first VR display system, and the existing virtual environment is a virtual environment rendered to a supervising user via a second VR display system in communication with the processor.
4. The system of embodiment 1, wherein generating or modifying the virtual environment comprises generating the virtual environment as a new virtual environment.
5. The system of any one of embodiments 1 to 4, wherein the customisation input is provided via an interaction with a GUI, optionally wherein the interaction is one or more of:
   an interaction made outside of the virtual environment,
   an interaction with an application running on a computing device,
   an interaction with a web form, or
   a selection from a menu.
6. The system of embodiment 3, wherein the customisation input is provided via an interaction of the supervising user with at least a component of the VR display system.
7. The system of any one of embodiments 1 to 6, wherein rendering the virtual environment to the training recipient comprises setting, based on the state of the virtual patient, an external appearance of the virtual patient.
8. The system of any one of embodiments 1 to 7, wherein rendering the virtual environment to the training recipient comprises setting, based on the state of the virtual patient, a behaviour of the virtual patient.
9. The system of embodiment 8, wherein the set behaviour is a behaviour of the virtual patient in response to a given interaction of the training recipient with the virtual patient.
10. The system of any one of embodiments 1 to 9, wherein rendering the virtual environment to the training recipient comprises setting, based on the state of the virtual patient, a behaviour of a virtual medical resource in the set of available virtual medical resources.
11. The system of embodiment 10, wherein the set behaviour is a behaviour of the virtual medical resource in response to a given interaction of the training recipient with the virtual patient.
12. The system of embodiment 9 or embodiment 11, wherein the given interaction comprises a use within the virtual environment, by the training recipient, upon the virtual patient, of a virtual medical resource in the set of available virtual medical resources.
13. The system of any one of embodiments 1 to 12, wherein the virtual environment further comprises a set of one or more available digital audio assets configured to be played in the virtual environment to the training recipient.
14. The system of embodiment 13, wherein rendering the virtual environment to the training recipient comprises causing, or enabling the training recipient to interact with the virtual environment to cause, one of the digital audio assets to be played in the virtual environment to the training recipient.
15. The system of any one of embodiments 1 to 14, wherein rendering the virtual environment to the training recipient comprises causing the display of one or more virtual medical resources of the set of available virtual medical resources in the 3D virtual scene.
16. The system of any one of embodiments 1 to 14, wherein rendering the virtual environment to the training recipient comprises enabling the training recipient to interact with the virtual environment to cause the display of one or more virtual medical resources of the set of available virtual medical resources in the 3D virtual scene.
17. The system of embodiments 16, wherein the interaction by the training recipient with the virtual environment is by means of the VR display system.
18. The system of any one of embodiments 15 to 17, wherein the displayed one or more virtual medical resources comprise one or more of:
   a virtual medical item,
   a virtual medical tool,
   a virtual display,
   a virtual consumable medical product,
   a virtual pharmaceutical product,
   a 2D media asset, or
   a text asset.
19. The system of any one of embodiments 15 to 18, wherein rendering the virtual environment to the training recipient comprises displaying, or enabling the training recipient to cause the display of, a 2D media asset or text asset via a virtual display in the 3D virtual scene.
20. The system of any one of embodiments 1 to 19, wherein the training recipient is a first training recipient, the VR display system is a first VR display system, and the instructions are further configured to cause the processor to:
   communicate with a third VR display system to render the generated or modified virtual environment to a second training recipient via said third VR display system.
21. The system of any one of embodiments 1 to 20, wherein the customisation input is a first customisation input received from a first supervising user, and the instructions are further configured to cause the processor to:
   receive a second customisation input from a second supervising user, wherein the second customisation input is a manual input received to customise the state of the virtual patient and/or the set of available virtual medical resources;
   modify the virtual environment based on the second customisation input; and
   communicate with the VR display system to render the generated or modified virtual environment to the training recipient via said VR display system.
22. The system of any one of embodiments 1 to 21, wherein the state of the virtual patient comprises one or more of:
   a body temperature of the virtual patient;
   a heart rate of the virtual patient;
   a respiration rate of the virtual patient;
   a blood pressure of the virtual patient;
   an oxygen saturation of the virtual patient;
   a blood glucose level of the virtual patient;
   a blood CO₂ level of the virtual patient;
   an end-tidal CO₂ level of the patient;
   an age of the virtual patient;
   a gender of the virtual patient;
   an ethnicity of the virtual patient;
   a position of the virtual patient;
   a clothing state of the virtual patient;
   a responsivity of the virtual patient;
   a pupil size of the virtual patient;
   a pupil reactivity of the virtual patient;
   a skeletal pathology of the virtual patient;
   a respiratory pathology of the virtual patient;
   a circulatory pathology of the virtual patient; or
   an audio asset associated with the virtual patient.
23. A VR display system comprising a processor and a memory, memory containing instructions which, when executed by the processor, cause the processor to:
   communicate with a customisable medical training system; and
   render, to a training recipient, a virtual environment generated or modified by the customisable medical training system based on a customisation input, the virtual environment comprising:
      a 3D virtual scene in which a virtual patient is displayed,
      a state of the virtual patient; and
      a set of one or more available virtual medical resources displayable in the 3D virtual scene.
24. A computer-implemented method of providing customised medical training, the method comprising:
   generating or modifying, by a processor and based on a customisation input, a virtual environment comprising:
      a 3D virtual scene in which a virtual patient is displayed,
      a state of the virtual patient; and
      a set of one or more available virtual medical resources displayable in the 3D virtual scene;
   communicating, by the processor, with a VR display system; and
   rendering to a training recipient, by the VR display system, the generated or modified virtual environment;
   wherein the customisation input is a manual input received to customise the state of the virtual patient and/or the set of available virtual medical resources.
25. A non-transitory computer-readable medium comprising instructions which, when read by a computer, cause the computer to:
   generate or modify, based on a customisation input, a virtual environment comprising:
      a 3D virtual scene in which a virtual patient is displayed,
      a state of the virtual patient; and
      a set of one or more available virtual medical resources displayable in the 3D virtual scene; and
   communicate with a VR display system to render the generated or modified virtual environment to a training recipient via said VR display system;
   wherein the customisation input is a manual input received to customise the state of the virtual patient and/or the set of available virtual medical resources.

## Claims

1. A customisable medical training system comprising a processor and a memory, the memory containing instructions which, when executed by the processor, cause the processor to:
generate or modify, based on a customisation input, a virtual environment comprising:
a 3D virtual scene in which a virtual patient is displayed,
a state of the virtual patient; and
a set of one or more available virtual medical resources displayable in the 3D virtual scene; and
communicate with a VR display system to render the generated or modified virtual environment to a training recipient via said VR display system;
wherein the customisation input is a manual input received to customise the state of the virtual patient and/or the set of available virtual medical resources.

2. The system of claim 1, wherein generating or modifying the virtual environment comprises modifying an existing virtual environment rendered to the training recipient, optionally wherein the VR display system is a first VR display system, and the existing virtual environment is a virtual environment rendered to a supervising user via a second VR display system in communication with the processor, optionally wherein the customisation input is provided via an interaction of the supervising user with at least a component of the VR display system.

3. The system of claim 1, wherein generating or modifying the virtual environment comprises generating the virtual environment as a new virtual environment.

4. The system of any one of claims 1 to 3, wherein the customisation input is provided via an interaction with a GUI, optionally wherein the interaction is one or more of:
an interaction made outside of the virtual environment,
an interaction with an application running on a computing device,
an interaction with a web form, or
a selection from a menu.

5. The system of any one of claims 1 to 4, wherein rendering the virtual environment to the training recipient comprises setting, based on the state of the virtual patient, either an external appearance of the virtual patient or a behaviour of the virtual patient, optionally wherein the set behaviour is a behaviour of the virtual patient in response to a given interaction of the training recipient with the virtual patient, optionally wherein the given interaction comprises a use within the virtual environment, by the training recipient, upon the virtual patient, of a virtual medical resource in the set of available virtual medical resources.

6. The system of any one of claims 1 to 5, wherein rendering the virtual environment to the training recipient comprises setting, based on the state of the virtual patient, a behaviour of a virtual medical resource in the set of available virtual medical resources, optionally wherein the set behaviour is a behaviour of the virtual medical resource in response to a given interaction of the training recipient with the virtual patient, optionally wherein the given interaction comprises a use within the virtual environment, by the training recipient, upon the virtual patient, of a virtual medical resource in the set of available virtual medical resources.

7. The system of any one of claims 1 to 6, wherein the virtual environment further comprises a set of one or more available digital audio assets configured to be played in the virtual environment to the training recipient, optionally wherein rendering the virtual environment to the training recipient comprises causing, or enabling the training recipient to interact with the virtual environment to cause, one of the digital audio assets to be played in the virtual environment to the training recipient.

8. The system of any one of claims 1 to 7, wherein rendering the virtual environment to the training recipient comprises either:
causing the display of one or more virtual medical resources of the set of available virtual medical resources in the 3D virtual scene; or
enabling the training recipient to interact with the virtual environment to cause the display of one or more virtual medical resources of the set of available virtual medical resources in the 3D virtual scene, optionally wherein the interaction by the training recipient with the virtual environment is by means of the VR display system,
optionally wherein rendering the virtual environment to the training recipient comprises displaying, or enabling the training recipient to cause the display of, a 2D media asset or text asset via a virtual display in the 3D virtual scene.

9. The system of claim 8, wherein the displayed one or more virtual medical resources comprise one or more of:
a virtual medical item,
a virtual medical tool,
a virtual display,
a virtual consumable medical product,
a virtual pharmaceutical product,
a 2D media asset, or
a text asset.

10. The system of any one of claims 1 to 9, wherein the training recipient is a first training recipient, the VR display system is a first VR display system, and the instructions are further configured to cause the processor to:
communicate with a third VR display system to render the generated or modified virtual environment to a second training recipient via said third VR display system.

11. The system of any one of claims 1 to 10, wherein the customisation input is a first customisation input received from a first supervising user, and the instructions are further configured to cause the processor to:
receive a second customisation input from a second supervising user, wherein the second customisation input is a manual input received to customise the state of the virtual patient and/or the set of available virtual medical resources;
modify the virtual environment based on the second customisation input; and
communicate with the VR display system to render the generated or modified virtual environment to the training recipient via said VR display system.

12. The system of any one of claims 1 to 11, wherein the state of the virtual patient comprises one or more of:
a body temperature of the virtual patient;
a heart rate of the virtual patient;
a respiration rate of the virtual patient;
a blood pressure of the virtual patient;
an oxygen saturation of the virtual patient;
a blood glucose level of the virtual patient;
a blood CO₂ level of the virtual patient;
an end-tidal CO₂ level of the patient;
an age of the virtual patient;
a gender of the virtual patient;
an ethnicity of the virtual patient;
a position of the virtual patient;
a clothing state of the virtual patient;
a responsivity of the virtual patient;
a pupil size of the virtual patient;
a pupil reactivity of the virtual patient;
a skeletal pathology of the virtual patient;
a respiratory pathology of the virtual patient;
a circulatory pathology of the virtual patient; or
an audio asset associated with the virtual patient.

13. A VR display system comprising a processor and a memory, memory containing instructions which, when executed by the processor, cause the processor to:
communicate with a customisable medical training system; and
render, to a training recipient, a virtual environment generated or modified by the customisable medical training system based on a customisation input, the virtual environment comprising:
a 3D virtual scene in which a virtual patient is displayed,
a state of the virtual patient; and
a set of one or more available virtual medical resources displayable in the 3D virtual scene.

14. A computer-implemented method of providing customised medical training, the method comprising:
generating or modifying, by a processor and based on a customisation input, a virtual environment comprising:
a 3D virtual scene in which a virtual patient is displayed,
a state of the virtual patient; and
a set of one or more available virtual medical resources displayable in the 3D virtual scene;
communicating, by the processor, with a VR display system; and
rendering to a training recipient, by the VR display system, the generated or modified virtual environment;
wherein the customisation input is a manual input received to customise the state of the virtual patient and/or the set of available virtual medical resources.

15. A non-transitory computer-readable medium comprising instructions which, when read by a computer, cause the computer to:
generate or modify, based on a customisation input, a virtual environment comprising:
a 3D virtual scene in which a virtual patient is displayed,
a state of the virtual patient; and
a set of one or more available virtual medical resources displayable in the 3D virtual scene; and
communicate with a VR display system to render the generated or modified virtual environment to a training recipient via said VR display system;
wherein the customisation input is a manual input received to customise the state of the virtual patient and/or the set of available virtual medical resources.
